(19) Europäisches Patentamt
European Patent Office
Office européen des brevets

(11) Veröffentlichungsnummer: **0 125 204**
**A1**

(12) # EUROPÄISCHE PATENTANMELDUNG

(21) Anmeldenummer: 84810171.3

(22) Anmeldetag: 06.04.84

(51) Int. Cl.³: C 07 C 43/29
C 07 C 43/267, C 07 C 121/75
A 01 N 31/08, A 01 N 37/38

(30) Priorität: 12.04.83 CH 1954/83

(43) Veröffentlichungstag der Anmeldung: 14.11.84 Patentblatt 84/46

(84) Benannte Vertragsstaaten: BE CH DE FR GB IT LI NL

(71) Anmelder: CIBA-GEIGY AG
Postfach
CH-4002 Basel(CH)

(72) Erfinder: Ackermann, Peter, Dr.
Hangelimattweg 113
CH-4148 Pfeffingen(CH)

(72) Erfinder: Gsell, Laurenz, Dr.
Maiengasse 56
CH-4056 Basel(CH)

(54) **3-Phenoxybenzyl-(2-phenyl-2,2-alkylen-äthyl)-äther und -thioäther, Verfahren zu ihrer Herstellung und ihre Verwendung in der Schädlingsbekämpfung.**

(57) 3-Phenoxybenzyl-(2-phenyl-2, 2-alkylen-äthyl) -äther der Formel

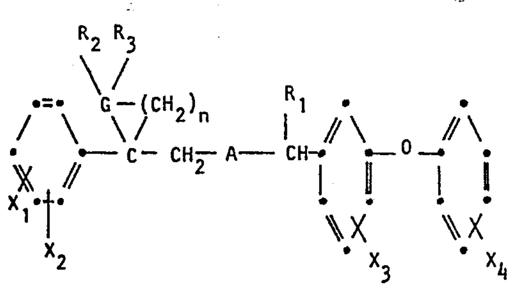

(I)

worin
A Sauerstoff oder Schwefel,
$R_1$ Wasserstoff, Methyl, Cyano oder Aethinyl,
$R_2$ und $R_3$ Wasserstoff, Halogen oder $C_1$-$C_5$-Alkyl,
n die Zahlen 1 bis 4,
$X_1$ Wasserstoff, Halogen, $C_1$-$C_5$-Alkyl, $C_1$-$C_5$-Halogenalkyl, $C_1$-$C_5$-Alkoxy oder $C_1$-$C_5$-Halogenalkoxy,
$X_2$ Wasserstoff, Halogen, $C_1$-$C_5$-Alkyl oder zusammen mit $X_1$ in Nachbarstellung Methylendioxy und
$X_3$ und $X_4$ je Wasserstoff oder Halogen
bedeuten.

Es werden ein Verfahren zur Herstellung dieser Verbindungen und ihre Verwendung in der Schädlingsbekämpfung beschrieben.

EP 0 125 204 A1

Croydon Printing Company Ltd

5-14384/1+2

3-Phenoxybenzyl-(2-phenyl-2,2-alkylen-äthyl)-äther und -thioäther, Verfahren zu ihrer Herstellung und ihre Verwendung in der Schädlingsbekämpfung

Die vorliegende Erfindung betrifft 3-Phenoxybenzyl-(2-phenyl-2,2-alkylen-äthyl)-äther und -thioäther, Verfahren zu ihrer Herstellung und ihre Verwendung in der Schädlingsbekämpfung.

Die 3-Phenoxybenzyl-(2-phenyl-2,2-alkylen-äthyl)-äther und -thioäther haben die Formel

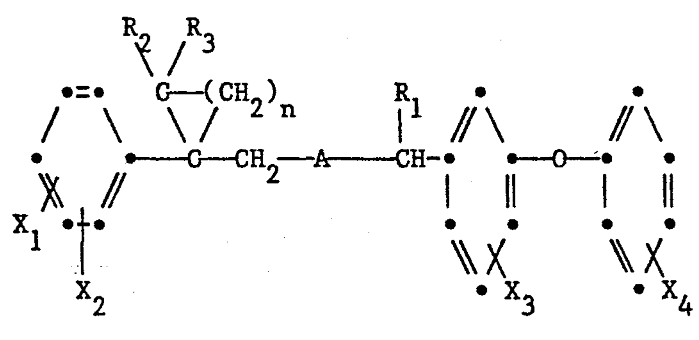

(I)

worin

A Sauerstoff oder Schwefel,

$R_1$ Wasserstoff, Methyl, Cyano oder Aethinyl,

$R_2$ und $R_3$ Wasserstoff, Halogen oder $C_1$-$C_5$-Alkyl,

n die Zahlen 1 bis 4,

$X_1$ Wasserstoff, Halogen, $C_1$-$C_5$-Alkyl, $C_1$-$C_5$-Halogenalkyl, $C_1$-$C_5$-Alkoxy oder $C_1$-$C_5$-Halogenalkoxy,

$X_2$ Wasserstoff, Halogen, $C_1$-$C_5$-Alkyl oder zusammen mit $X_1$ in Nachbarstellung Methylendioxy und

$X_3$ und $X_4$ je Wasserstoff oder Halogen

bedeuten.

Unter Halogen ist dabei Fluor, Chlor, Brom oder Jod, insbesondere aber Fluor oder Chlor, zu verstehen.

Die Alkyl-, Halogenalkyl-, Alkoxy- und Halogenalkoxygruppen bei $R_2$, $R_3$, $X_1$ und $X_2$ können geradkettig oder verzweigt sein. Beispiele solcher Gruppen sind u.a.: Methyl, Trifluormethyl, Methoxy, Difluormethoxy, Aethyl, Aethoxy, Propyl, Isopropyl, n-Butyl, n-Pentyl und deren Isomere.

Bevorzugt sind Verbindungen der Formel I, worin

A Sauerstoff oder Schwefel,

$R_1$ Wasserstoff, Methyl, Cyano oder Aethinyl,

$R_2$ und $R_3$ je Wasserstoff, Halogen oder $C_1$-$C_5$-Alkyl,

n die Zahlen 1 bis 4,

$X_1$ Wasserstoff, Halogen, $C_1$-$C_5$-Alkyl, $C_1$-$C_5$-Halogenalkyl, $C_1$-$C_5$-Alkoxy oder $C_1$-$C_5$-Halogenalkoxy,

$X_2$ Wasserstoff, Halogen oder $C_1$-$C_5$-Alkyl und

$X_3$ und $X_4$ je Wasserstoff oder Halogen bedeuten,

mit der Massgabe, dass $R_2$ und $R_3$ nicht gleichzeitig Wasserstoff oder Fluor sein können, falls A Sauerstoff, $R_1$ Wasserstoff und n die Zahl 1 bedeuten.

Besonders bevorzugt sind Verbindungen der Formel I, worin

A Sauerstoff oder Schwefel,

$R_1$ Wasserstoff,

$R_2$ und $R_3$ je Wasserstoff, Halogen oder Methyl,

n die Zahlen 1 bis 4,

$X_1$ Wasserstoff, Halogen, $C_1$-$C_4$-Alkyl, $C_1$-$C_4$-Alkoxy, Trifluormethyl oder Difluormethoxy,

$X_2$ Wasserstoff und

$X_3$ und $X_4$ je Wasserstoff oder Halogen bedeuten, mit der Massgabe, dass $R_2$ und $R_3$ nicht gleichzeitig Wasserstoff oder Fluor sein können, falls A Sauerstoff und n die Zahl 1 bedeuten.

Insbesondere bevorzugt sind Verbindungen der Formel I, worin

A Sauerstoff,

$R_1$ Wasserstoff,

$R_2$ und $R_3$ je Wasserstoff, Halogen oder Methyl,

n die Zahlen 2 bis 4,

$X_1$ Wasserstoff, Halogen, Methoxy oder Aethoxy,

$X_2$ Wasserstoff und

$X_3$ und $X_4$ je Wasserstoff oder Halogen bedeuten.

Die Verbindungen der Formel I werden nach an sich bekannten Methoden, z.B. wie folgt hergestellt:

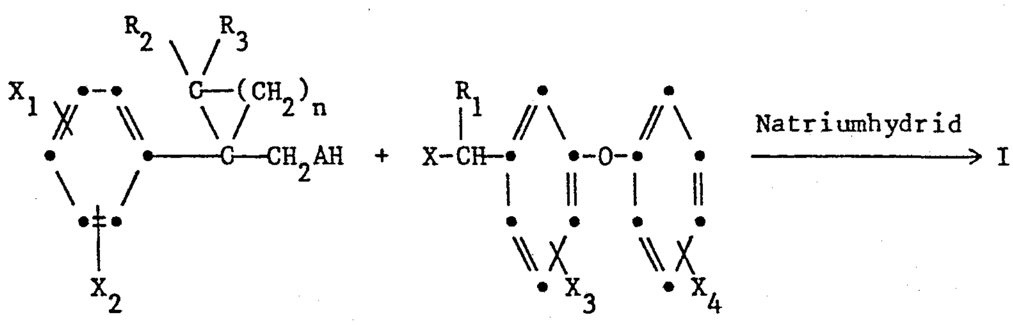


(II) (III)

In den Formeln II und III haben A, $R_1$, $R_2$, $R_3$, n und $X_1$-$X_4$ die für die Formel I angegebene Bedeutung.

In der Formel III steht X für ein Halogenatom insbesondere für Chlor oder Brom oder die p-Toluolsulfonatgruppe.

Das Verfahren wird bei einer Reaktionstemperatur zwischen -10 und 120°C, meist zwischen 20 und 80°C, bei normalem oder erhöhtem Druck und vorzugsweise in einem inerten Lösungs- oder Verdünnungsmittel

durchgeführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Acetone und Methylketon sowie Hexan.

Die Ausgangsstoffe der Formeln II und III sind bekannt oder können analog bekannten Methoden hergestellt werden.

Die Verbindungen der Formel I liegen als Gemische von verschiedenen optisch aktiven Isomeren vor, wenn bei der Herstellung nicht einheitlich optisch aktive Ausgangsmaterialen verwendet werden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen Schädlingen an Tieren und Pflanzen. So können sie zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwoll- und Reiskulturen (z.B. Spodoptera littoralis, Heliothis virescens, Nilaparvata lugens, Chilo suppressalis und Laodelphax) sowie Gemüse- und Obstkulturen (z.B. Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savigni und Scotia ypsilon).

Wirkstoffe der Formel I zeigen auch eine sehr günstige Wirkung gegen Fliegen, wie z.B. Musca domestica sowie Mückenlarven und gegen ektoparasitäre Milben und Zecken, z.B. der Familien Ixodidae, Argasidae und Dermanyssidae. Daneben zeichnen sich die Verbindungen der Formel I durch eine breite ovizide und ovolarvizide Wirkung aus.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propionyläther, Propinyloxime, Propinylcarbamate und Propinylphosphonate, 2-(3,4-Methylendioxy-phenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahen wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22-C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

> "McCutcheon's Detergents and Emulsifiers Annual" MC Publishing Corp., Ringwood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel Haftmittel sowie Dünger oder andere Wirkstoffe zur Erzielung spezieller Effekte enthalten.

Formulierungsbeispiele für flüssige Wirkstoffe der Formel I

(% = Gewichtsprozent)

| 1. Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthyklenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80 % | 10 % | 5 % | 95 % |
| Aethylenglykol-monomethyläther | 20 | - | - | - |
| Polyäthylenglykol M.G. 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) |
|---|---|---|
| Wirkstoff | 5 % | 10 % |
| Kaolin | 94% | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) | c) |
|---|---|---|---|
| Wirkstoff | 20 % | 50 % | 75 % |
| Na-Ligninsulfonat | 5 % | 5 % | - |
| Na-Laurylsulfat | 3 % | - | 5% |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67% | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3 % |
| Polyäthylenglykol (M.G. 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1% |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung der Verbindung Nr. 1 der Formel

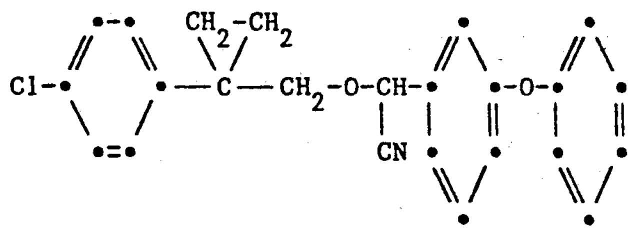

Zu einer auf 95°C erhitzten Mischung von 1,35 g Natriumhydrid und 80 ml Dimethylformamid/Toluol (1:1) wird innert 15 Minuten eine Lösung von 4,1 g der Verbindung der Formel

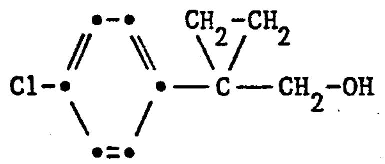

in 5 ml Dimethylformamid/Toluol (1:1) zugetropft. Nach 15 minütigem Rühren bei 95°C wird das Reaktionsgemisch auf 50°C abgekühlt, innert 3 Stunden eine Lösung von 7,7 g der Verbindung der Formel

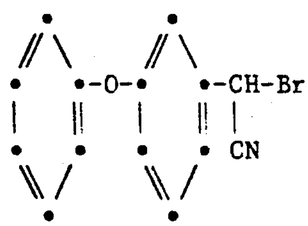

in 10 ml Dimethylformamid/Toluol (1:1) zugetropft und anschliessend 1,35 g KJ und 0,2 g 18-Crown-6-äther zugegeben. Das Reaktionsgemisch wird 24 Stunden bei 20°C stehengelassen, mit gesättigter Ammoniumchloridlösung versetzt und mit Aether extrahiert. Die Aetherphase wird mit gesättigter Kochsalzlösung gewaschen, über Magnesiumsulfat getrocknet und eingeengt. Nach dem Chromatographieren des Produktes an Kieselgel mit Hexan/Aether (5:1) als Eluiermittel erhält man die Titelverbindung mit einer Refraktion von $n_D^{20°} = 1,5923$.

Auf analoge Weise werden auch folgende Verbindungen der Formel I, worin A gleich Sauerstoff, hergestellt:

| Nr. | $X_1$ para | $X_2$ | $X_3$ | $X_4$ | $R_1$ | $R_2$ | $R_3$ | n | Refraktion |
|---|---|---|---|---|---|---|---|---|---|
| 2 | t-$C_4H_9$ | H | H | H | H | H | H | 1 | $n_D^{21°}$ =1,5693 |
| 3 | Cl | H | H | H | H | H | H | 1 | $n_D^{20°}$ =1,5899 |
| 4 | EtO | H | H | H | H | H | H | 1 | $n_D^{24°}$ =1,5762 |
| 5 | MeO | H | H | H | H | H | H | 1 | $n_D^{22°}$ =1,5828 |
| 6 | MeO | H | 4-F | H | H | H | H | 1 | $n_D^{22°}$ =1,5744 |
| 7 | H | H | H | H | H | Cl | Cl | 1 | $n_D^{22°}$ =1,5919 |
| 8 | Cl | H | H | H | H | Cl | Cl | 1 | $n_D^{22°}$ =1,5936 |
| 9 | Cl | H | H | H | H | H | H | 2 | $n_D^{25°}$ =1,5850 |
| 10 | Cl | H | 4-F | H | H | H | H | 2 | $n_D^{24°}$ =1,5764 |
| 11 | H | H | 4-F | H | H | $CH_3$ | H | 2 | $n_D^{20°}$ =1,5654 |
| 12 | H | H | H | H | H | $CH_3$ | H | 2 | $n_D^{30°}$ =1,5756 |
| 13 | Cl | H | H | 4-F | H | H | H | 1 | $n_D^{20°}$ =1,5790 |
| 14 | Cl | H | H | 4-Br | H | H | H | 1 | $n_D^{20°}$ =1,6043 |
| 15 | Cl | H | 4-F | H | H | H | H | 1 | $n_D^{20°}$ =1,5788 |
| 16 | Br | H | H | H | H | $CH_3$ | H | 2 | $n_D^{21°}$ =1,5910 |
| 17 | Br | H | 4-F | H | H | $CH_3$ | H | 2 | $n_D^{21°}$ =1,5826 |
| 18 | MeO | H | H | 4-F | H | H | H | 4 | $n_D^{22°}$ =1,5696 |
| 19 | MeO | H | 4-F | H | H | H | H | 4 | $n_D^{22°}$ =1,5729 |
| 20 | MeO | H | H | 4-F | H | H | H | 3 | $n_D^{22°}$ =1,5714 |
| 21 | MeO | H | H | 4-F | H | H | H | 1 | $n_D^{21°}$ =1,5720 |
| 22 | EtO | H | H | 4-Br | H | H | H | 1 | $n_D^{21°}$ =1,5844 |

| Nr. | $X_1$ para | $X_2$ | $X_3$ | $X_4$ | $R_1$ | $R_2$ | $R_3$ | n | Refraktion |
|---|---|---|---|---|---|---|---|---|---|
| 23 | MeO | H | H | H | H | H | H | 4 | $n_D^{21^\circ}$ =1,5821 |
| 24 | MeO | H | H | 4-Cl | H | H | H | 3 | $n_D^{22^\circ}$ =1,5825 |
| 25 | EtO | H | H | 4-Cl | H | H | H | 3 | $n_D^{22^\circ}$ =1,5774 |
| 26 | EtO | H | H | 4-Br | H | H | H | 3 | $n_D^{22^\circ}$ =1,5868 |
| 27 | EtO | H | H | H | H | H | H | 3 | $n_D^{22^\circ}$ =1,5763 |
| 28 | MeO | H | H | 4-Br | H | H | H | 4 | $n_D^{22^\circ}$ =1,5911 |
| 29 | Cl | H | H | 4-Cl | H | H | H | 1 | $n_D^{26^\circ}$ =1,5935 |
| 30 | Cl | H | H | 4-F | H | H | H | 3 | $n_D^{26^\circ}$ =1,5753 |
| 31 | EtO | H | H | 4-Cl | H | H | H | 1 | $n_D^{24^\circ}$ =1,5814 |
| 32 | EtO | H | H | 4-F | H | H | H | 3 | $n_D^{25^\circ}$ =1,5650 |
| 33 | Cl | H | H | H | H | H | H | 3 | $n_D^{25^\circ}$ =1,5833 |
| 34 | Cl | H | H | 4-Br | H | H | H | 3 | Harz |
| 35 | MeO | H | H | 4-Br | H | H | H | 3 | $n_D^{22^\circ}$ =1,5953 |
| 36 | Cl | H | H | 4-Cl | H | H | H | 3 | $n_D^{23^\circ}$ =1,5918 |
| 37 | Cl | H | H | H | H | H | H | 4 | $n_D^{22^\circ}$ =1,5782 |
| | sowie die Isomeren der Formel | | | | | | | | |
| 38 | 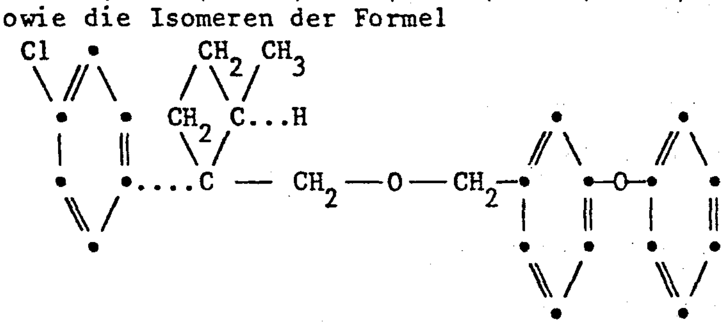 | | | | | | | | $n_D^{24^\circ}$ =1,5750 |
| 39 | 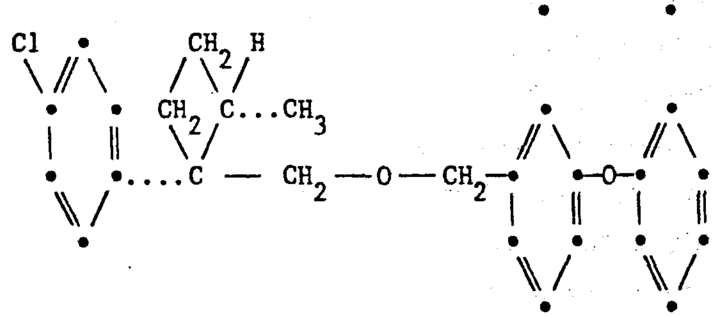 | | | | | | | | $n_D^{22^\circ}$ =1,5810 |

Beispiel 2: Insektizide Frassgift-Wirkung: Spodoptera littoralis

Baumwollpflanzen werden mit einer Versuchslösung, enthaltend 400 ppm der zu prüfenden Verbindung, besprüht.

Nach dem Antrocknen des Belages werden die Pflanzen mit Larven von Spodoptera littoralis (L3-Stadium) besetzt. Man verwendet pro Versuchsverbindung und pro Test-Spezies zwei Pflanzen. Die Auswertung der erzielten Abtötungsrate erfolgt nach 2, 4, 24 und 48 Stunden. Der Versuch wird bei 28°C und 60 % relativer Luftfeuchtigkeit durchgeführt.

Verbindungen gemäss Beispiel 1 zeigen im obigen Test 100%ige Wirkung gegenüber Spodoptera littoralis Larven.

Beispiel 3: Wirkung gegen Diabrotica balteata

750 ml Komposterde werden mit 150 ml Testlösung enthaltend 3 ppm Wirkstoff gemischt. Mit der behandelten Erde werden Maiskeimlinge in Plastiktöpfe eingetopft (4 Keimlinge pro Topf mit einem Durchmesser von 10 cm). Die Töpfe werden unmittelbar nachher mit 10 $L_3$-Larven von Diabrotica balteata infestiert. Die Kontrolle wird 10 Tage nach dem Einsetzen der Larven durchgeführt.

Verbindungen gemäss Beispiel 1 zeigen im obigen Test 100 %ige Wirkung gegen $L_3$-Larven von Diabrotica balteata.

Beispiel 4: Insektizide Frassgift-Wirkung: Nilaparvata lugens

Reispflanzen werden mit einer Versuchslösung, enthaltend 50 ppm der zu prüfenden Verbindung, besprüht. Nach dem Antrocknen des Belages werden die Pflanzen mit Larven von Nilaparvata lugens (L3-Stadium) besetzt. Man verwendet pro Versuchsverbindung und pro Test-Spezies zwei Pflanzen. Die Auswertung der erzielten Abtötungsrate erfolgt nach 24 Stunden. Der Versuch wird bei 22°C und 60 % relativer Luftfeuchtigkeit durchgeführt.

Verbindungen gemäss Beispiel 1 zeigen im obigen Test 100 % Wirkung gegenüber Nilaparvata lugens Larven.

Patentansprüche

1. Ein 3-Phenoxybenzyl-(2-phenyl-2,2-alkylen-äthyl)-äther der Formel

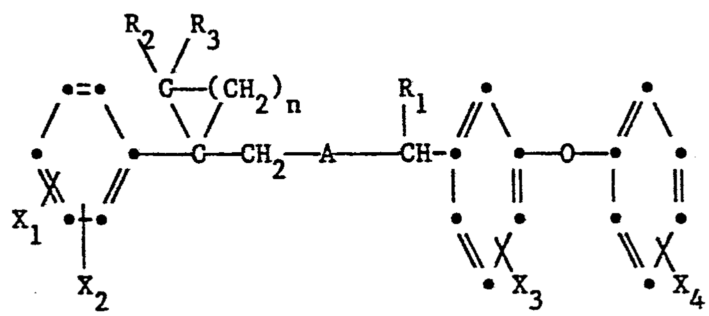

(I)

worin

A Sauerstoff oder Schwefel,

$R_1$ Wasserstoff, Methyl, Cyano oder Aethinyl,

$R_2$ und $R_3$ Wasserstoff, Halogen oder $C_1$-$C_5$-Alkyl,

n die Zahlen 1 bis 4,

$X_1$ Wasserstoff, Halogen, $C_1$-$C_5$-Alkyl, $C_1$-$C_5$-Halogenalkyl, $C_1$-$C_5$-Alkoxy oder $C_1$-$C_5$-Halogenalkoxy,

$X_2$ Wasserstoff, Halogen, $C_1$-$C_5$-Alkyl oder zusammen mit $X_1$ in Nachbarstellung Methylendioxy und

$X_3$ und $X_4$ je Wasserstoff oder Halogen

bedeuten.

2. Eine Verbindung gemäss Anspruch 1, worin

A Sauerstoff oder Schwefel,

$R_1$ Wasserstoff, Methyl, Cyano oder Aethinyl,

$R_2$ und $R_3$ je Wasserstoff, Halogen oder $C_1$-$C_5$-Alkyl,

n die Zahlen 1 bis 4,

$X_1$ Wasserstoff, Halogen, $C_1$-$C_5$-Alkyl, $C_1$-$C_5$-Halogenalkyl, $C_1$-$C_5$-Alkoxy oder $C_1$-$C_5$-Halogenalkoxy,

$X_2$ Wasserstoff, Halogen oder $C_1$-$C_5$-Alkyl und

$X_3$ und $X_4$ je Wasserstoff oder Halogen bedeuten,

mit der Massgabe, dass $R_2$ und $R_3$ nicht gleichzeitig Wasserstoff oder Fluor sein können, falls A Sauerstoff, $R_1$ Wasserstoff und n die Zahl 1 bedeuten.

3. Eine Verbindung gemäss Anspruch 2, worin
A Sauerstoff oder Schwefel,
$R_1$ Wasserstoff,
$R_2$ und $R_3$ je Wasserstoff, Halogen oder Methyl,
n die Zahlen 1 bis 4,
$X_1$ Wasserstoff, Halogen, $C_1$-$C_4$-Alkyl, $C_1$-$C_4$-Alkoxy, Trifluormethyl oder Difluormethoxy,
$X_2$ Wasserstoff und
$X_3$ und $X_4$ je Wasserstoff oder Halogen
bedeuten, mit der Massgabe, dass $R_2$ und $R_3$ nicht gleichzeitig Wasserstoff oder Fluor sein können, falls A Sauerstoff und n die Zahl 1 bedeuten.

4. Eine Verbindung gemäss Anspruch 3, worin
A Sauerstoff,
$R_1$ Wasserstoff,
$R_2$ und $R_3$ je Wasserstoff, Halogen oder Methyl,
n die Zahlen 2 bis 4,
$X_1$ Wasserstoff, Halogen, Methoxy oder Aethoxy,
$X_2$ Wasserstoff und
$X_3$ und $X_4$ je Wasserstoff oder Halogen
bedeuten.

5. Die Verbindung gemäss Anspruch 1 der Formel

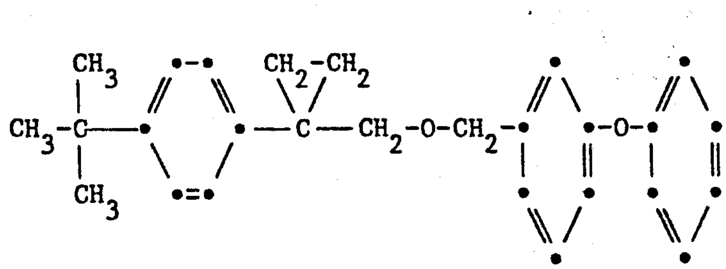

.

6. Die Verbindung gemäss Anspruch 1 der Formel

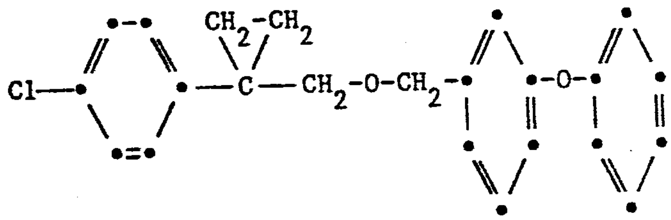

7. Die Verbindung gemäss Anspruch 1 der Formel

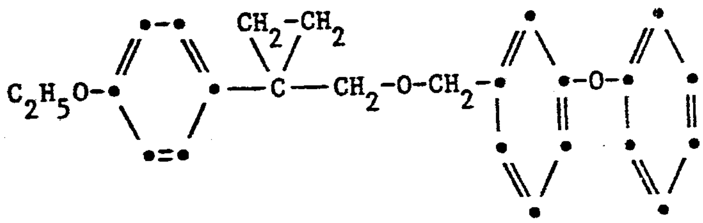

8. Die Verbindung gemäss Anspruch 1 der Formel

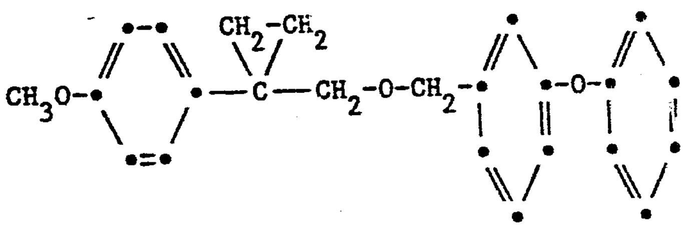

9. Die Verbindung gemäss Anspruch 1 der Formel

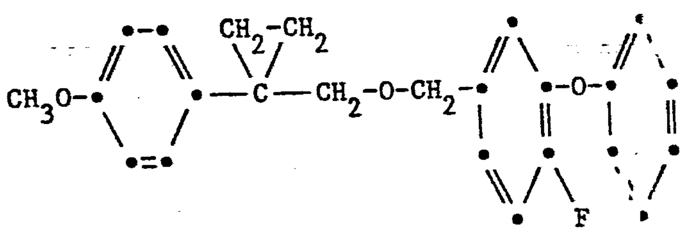

10. Die Verbindung gemäss Anspruch 3 der Formel

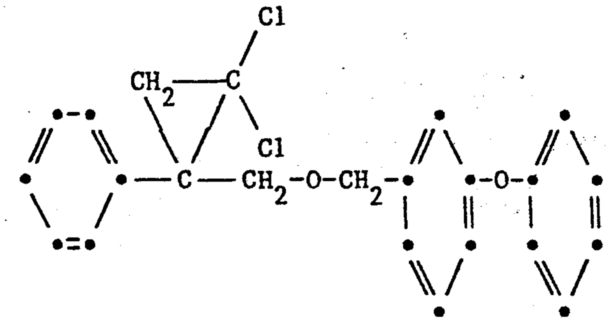

11. Die Verbindung gemäss Anspruch 3 der Formel

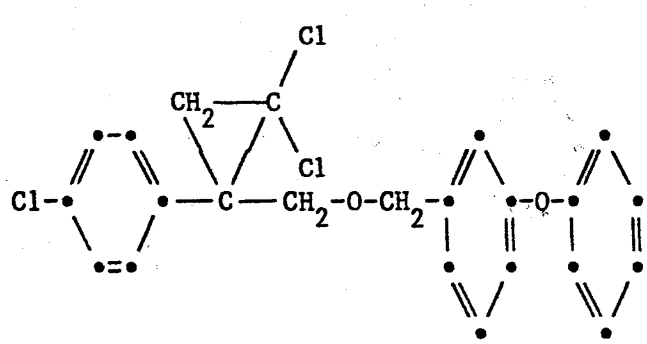

12. Die Verbindung gemäss Anspruch 2 der Formel

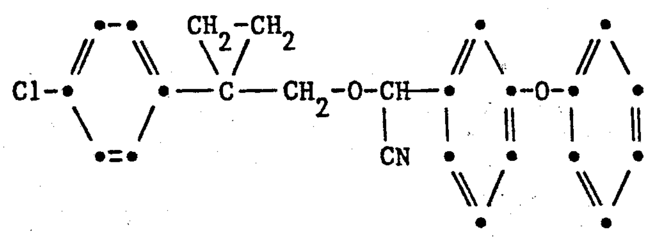

13. Die Verbindung gemäss Anspruch 4 der Formel

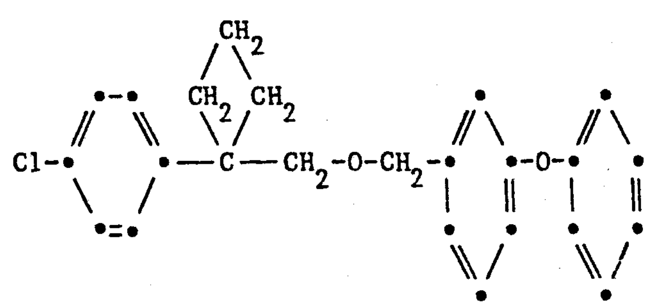

14. Die Verbindung gemäss Anspruch 4 der Formel

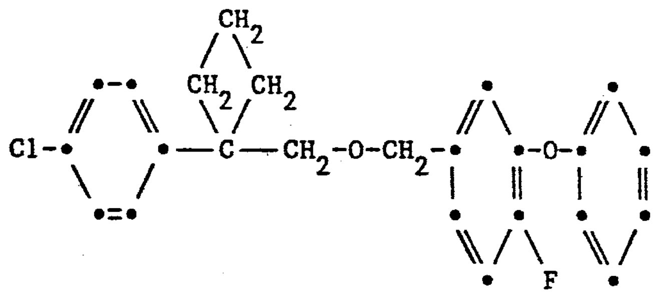

15. Die Verbindung gemäss Anspruch 4 der Formel

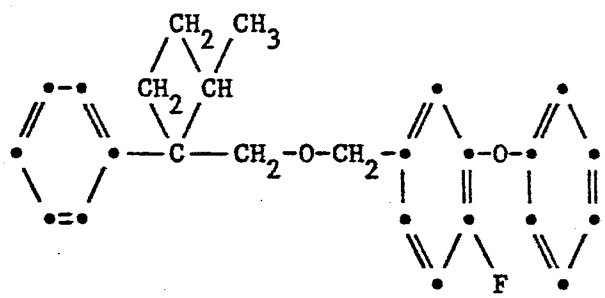

16. Die Verbindung gemäss Anspruch 4 der Formel

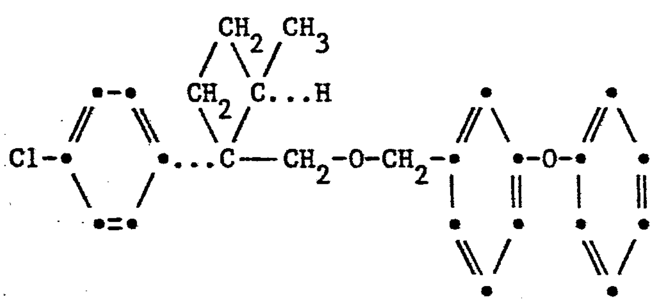

17. Die Verbindung gemäss Ansprch 4 der Formel

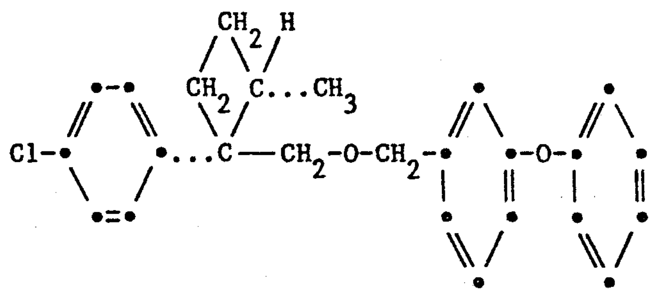

.

18. Ein Schädlingsbekämpfungsmittel, welches als aktive Komponente eine Verbindung gemäss Anspruch 1 und in der Formulierungstechnik übliche Hilfsmittel enthält.

19. Die Verwendung einer Verbindung gemäss Anspruch 1 zur Bekämpfung von verschiedenartigen Schädlingen an Tieren und Pflanzen.

20. Die Verwendung gemäss Anspruch 19 zur Bekämpfung von Insekten und Vertretern der Ordnung Akarina.

21. Verfahren zur Bekämpfung von verschiedenartigen Schädlingen an Tieren und Pflanzen, dadurch gekennzeichnet, dass man eine Verbindung gemäss Anspruch 1 auf die Tiere und Pflanzen appliziert.

FO 7.5 WH/jt*/eg*

Europäisches Patentamt

# EUROPÄISCHER RECHERCHENBERICHT

0125204

Nummer der Anmeldung

EP 84 81 0171

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-2 342 953 (SHELL INTERNATIONALE RESEARCH) * Ansprüche; Tabelle I; Beispiele 7,8,18 * | 1-21 | C 07 C 43/29<br>C 07 C 43/267<br>C 07 C 121/75<br>A 01 N 31/08<br>A 01 N 37/38 |
| A | GB-A-2 085 006 (MITSUI TOATSU CHEMICALS) * Ansprüche * | 1-21 | |
| A | AU-B- 502 950 (COMMONWEALTH SCIENTIFIC AND INDUSTRIAL RESEARCH ORGANIZATION) * Ansprüche * | 1-21 | |
| P,X | EP-A-0 094 085 (SUMITOMO) * Ansprüche; Tabelle 1, Verbindungen 1,3,5 * | 1,5-7, 18-21 | RECHERCHIERTE SACHGEBIETE (Int. Cl. 3) |
| P,X | EP-A-0 104 908 (COMMONWEALTH SCIENTIFIC AND INDUSTRIAL ORGANIZATION) * Ansprüche; Tabelle 1; Beispiele 1,3-8,15 * | 1-3,18 -21 | C 07 C 43/00 |

Der vorliegende Recherchenbericht wurde für alle Patentansprüche erstellt.

| Recherchenort | Abschlußdatum der Recherche | Prüfer |
|---|---|---|
| DEN HAAG | 20-07-1984 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument